# EUROPEAN PATENT APPLICATION

(11) **EP 1 016 718 A1**
(43) Date of publication of application: **05.07.2000**
(21) Application number: 98937801.3
(22) Date of filing: 12.08.1998
(51) Int. Cl.: C12N 15/12, C12N 15/63, C07K 14/435, C07K 16/18, C12Q 1/68, C12N 1/21, C12P 21/02, G01N 33/53

(54) **PROTEIN HAVING Zn FINGER-LIKE MOTIF**

(30) Priority: 13.08.1997 JP 23170497
(71) Applicant: Chugai Research Institute for Molecular Medicine Inc., Niihari-gun, Ibaraki 300-4101 (JP)
(72) Inventor: NEZU, Jun-ichi;-Chugai Res. Inst. for Molec. Med., 153-2, Nagai;,Niihari-mura, Niihari-gun (JP); OKU, Asuka;-Chugai Research Inst. for Molec. Med., 153-2, Nagai;,Niihari-mura, Niihari (JP)
(74) Representative: VOSSIUS & PARTNER
(86) International application number: JP9803601
(87) International publication number: WO9909158

(57) **Abstract**

A gene encoding a novel protein having a Zn finger-like motif, which has been succssfully isolated from cDNAs originating in human fetal brain by preparing primers based on an EST sequence retrieved by using the information of domains seemingly playing important functional roles in the TRAF (TNF receptor associated factor) family and employing these primers.

## Description

### Technical Field

The present invention relates to a novel polypeptide having the Zn finger-like motif and a gene encoding it.

### Background Art

TNF (tumor necrosis factor) was discovered as a cytokine with biological activity to induce cell death in some tumor cells (Pennica, D. et al. (1984) Nature, 312, 724-729). Subsequent research has demonstrated that TNF has diverse biological activities including immunomodulator activities to induce inflammatory responses (Beutler, B. (1992) Tumor Necrosis Factors: The Molecules and Their Emerging Role in Medicine, Raven Press, New York). Its diverse activities are based on a complicated signal transduction cascade, and the detail mechanisms are now being clarified (Tartalia, L. A., and Goeddel, D. V. (1992) Immunol. Today 13, 151-153; Rothe, J. et al. (1992) Immunol. Res. 11, 81-90).

Binding of TNF to its specific receptors (TNF-R1, TNF-R2) initiates signalings, which are mainly divided into two categories. One of them, "death" signal or apoptosis signal is transduced by TRAD (Hsu, H. et al. (1995) Cell 81, 495-504), RIP (Hsu, H. et al. (1996) Immunity 4, 387-396), and FADD/MORT1 (Boldin, M. P. et al. (1995) J. Biol. Chem. 270, 7795-7798; Chinnaiyan, A. M. et al. (1995) Cell 81, 505-512) and the like, which are directly or indirectly bound to the TNF receptors in cells. These factors form complexes by binding to each other via homologous regions called "death domain" consisting of about 80 amino acid residues, resulting in transduction of signals (Hsu, H. et al. (1996) Cell 84, 299-308). FADD is considered to bind to a polypeptide called caspase 8/FLICE/MACH (Boldin, M. P. et al. (1996) Cell 85, 803-815; Muzio, M. et al. (1996) Cell 85, 817-827; Alnemri, E. S. et al. (1996) Cell 87, 171) belonging to the ICE protease family and activates the ICE protease cascade downstream thereof to induce apoptosis.

TNF receptors also bind to a polypeptide belonging to a family called the TRAF (TNF receptor associated factor) family, leading to transduce a different signal from "death" signal transduced by factors of FADD system. TRAF is a factor that transduces "survival" signal by activating transcription factor NF-κB to promote transcription of target genes of NF-κB, thereby inducing immune responses and anti-apoptotic functions (Rothe, M, et al. (1994) cell 78, 681-692; Liu, Z. -G. et al. (1996) Cell 87, 565-576; Beg, A. A. et al. (1996) Science 274, 782-784; van Antwerp, D. J. et al. (1996) Science 274, 787-789; Wang, CC. -Y. et al. (1996) Science 274, 784-787). Thus, many factors are involved in the signal transduction through the TNF receptors and form very complicated cascade (Nagata, S. (1997) cell 88, 355-365).

A family of TNF (TNF family) includes, in addition to TNF, lymphotoxin (LT)α, LTβ, Fas ligand, CD30 ligand, 4-1BB ligand, CD27 ligand, TRAIL, and CD40 Ligand. A family of the TNF receptors includes LTβ receptor, the low-affinity nerve growth factor (NGF) receptor, Fas, CD30, CD27, and CD40. It is known that factors involved in TNF signaling are commonly used in a part of signal transduction of polypeptides of these families (Chinnaiyan, A. M. et al. (1996) Science 274, 990-992; Kiston, J. et al. (1996) Nature 384, 372-375), but much remains unknown. Although TNF and Fas ligand signalings have been rather elucidated, their whole picture has not been completely revealed, and there would be unidentified important factors.

A group of polypeptides called the IAP family is anticipated to relate to signal transduction of the TNF receptor. Polypeptides called cIAP-1 (HIAP-1), cIAP-2 (HIAP-2), NAIP, XIAP, DIAP, Cp-IAP, and Op-IAP belong to this family and commonly have conserved amino acid sequence named the BIR motif. cIAP-1 and cIAP-2 were originally identified as polypeptides in a complex that binds to the TNF receptor II (p75) cytoplasmic region. It was clarified later that they bind to the receptor through TRAF (Cell 83, 1243-1252, 1995). NAIP was identified by a positional cloning technique as a highly-frequently mutated gene in childhood spinal muscular atrophies (SMA), autosomal recessive disorder (Cell 80, 167-178, 1995). XIAP and DIP were identified as genes with homology to NAIP, and are considered to form a new family with above cIAP-1 and cIAP-2, and baculoviral polypeptides, Cp-IAP and Op-IAP. Cp-IAP and Op-IAP inhibit apoptosis in host cells of baculovirus. In addition, overexpression of other homologous genes in the IAP family is demonstrated to inhibit apoptosis induced by various stimuli (Nature 379, 349-353, 1996). The stimuli include stimulation caused by TNF. Taken together with the fact that cIAP-1 and cIAP-2 exist in a complex bound to the TNF-receptor, the IAP family must play an important role in signal transduction of the TNF family.

Recently, a polypeptide called XIAP associated factor-1 (XAF-1) that binds to XIAP, one of the IAP family has been registered in database (GenBank accession No. X99699). Because of binding to XIAP, XAF-1 is considered to be a polypeptide involved in not only exerting function of XIAP such as ability to inhibit apoptosis, but also exerting function of TNF.

Interestingly, a highly homologous region to the Zn finger motif in the TRAF family exists in the N-terminal amino acid sequence of XAF-1.

The Zn finger motif is well known as a sequence with DNA binding capacity characteristic of transcription factors. In addition, the Zn finger motif is presumably involved in protein-protein interaction (Science 272, 1797-1802, 1996). Functions of the Zn finger motif of the TRF family polypeptide and XAF-1 have not been clarified. However, the Zn finger motif must be important to construct specific three-dimensional structure via Zn, and play an important role in exerting functions of the polypeptide.

### Disclosure of the Invention

An objective of the present invention is to provide novel polypeptides involved in signal transduction of the TNF family and their genes. Specifically, an objective of the present invention is to provide polypeptides having the Zn finger motif and their genes.

The present inventors searched for a new factor involved in signal transduction cascade of the TNF family. The inventors focused on the Zn finger domain, which presumably plays a functionally important role in the TNF receptor associated factor (TRAF) family (Cao, Z., Xiong, J., Takeuchi, M., Kurama, T., and Goeddel, D.V. (1996) Nature 383, 443-446), and searched for a new gene in Expressed Sequence Tag (EST) database based on information about the Zn finger domain amino acid sequence of the TRAF family polypeptides. As a result, the inventors found a partial sequence significantly homologous to the Zn finger domain. Furthermore, the inventors isolated cDNA encoding an entire open reading frame using information of the partial sequence and analyzed its structure, and found that the isolated gene was an unknown gene encoding a new polypeptide with high homology to the Zn finger domain of the TRAF family polypeptides and XAF-1 identified recently.

The present invention relates to a novel polypeptide having a Zn finger-like motif and being presumably involved in the signal transduction cascade of the TNF family and its gene, more specifically,
(1) a polypeptide comprising the amino acid sequence set forth in SEQ ID NO:1, or a polypeptide comprising said sequence in which one or more amino acids are substituted, deleted, or added, and having substantially the same functions as the polypeptide comprising the amino acid sequence set forth in SEQ ID NO:1;
(2) a polypeptide encoded by DNA that hybridizes with DNA set forth in SEQ ID NO:2 and having substantially the same functions as a polypeptide comprising the amino acid sequence set forth in SEQ ID NO:1;
(3) a DNA encoding the polypeptide described in (1);
(4) a DNA hybridizing with the DNA set forth in SEQ ID NO:2 and encoding a polypeptide having substantially the same functions as a polypeptide comprising the amino acid sequence set forth in SEQ ID NO:1;
(5) a vector comprising the DNA described in (3) or (4);
(6) a transformant harboring the DNA described in (3) or (4);
(7) a method of producing the polypeptide described in (1) or (2); the method comprising culturing the transformant described in (6);
(8) an antibody that binds to the polypeptide described in (1) or (2);
(9) a method of screening for a compound that binds to the polypeptide described in (1) or (2), the method comprising contacting a test compound with the polypeptide described in (1) or (2) and selecting a compound bound to the polypeptide described in (1) or (2);
(10) the method described in (9), wherein a compound is a polypeptide;
(11) a method of screening for a gene encoding a polypeptide that binds to the polypeptide described in (1) or (2), the method comprising contacting a test gene product with the polypeptide described in (1) or (2), and selecting a gene corresponding to a gene product that binds to the polypeptide described in (1) or (2);
(12) a compound that binds to the polypeptide described in (1) or (2);
(13) the compound described in (12), wherein the compound is a polypeptide;
(14) the compound described in (12), wherein the compound is isolable by the method described in (9);
(15) the compound described in (13), wherein the compound is isolable by the method described in (10);
(16) a gene encoding a polypeptide that binds to the polypeptide described in (1) or (2); and
(17) the gene described in (16), wherein the gene is isolable by the method described in (11).

The "Zn finger-like motif" used herein indicates "Cys-Xaa(2)-Cys-Xaa(11)-His-Xaa(3 or 5)-Cys (Xaa represents any amino acid)."

The present invention relates to a novel polypeptide having the Zn finger-like motif. The fln29 polypeptide (SEQ ID NO:1) included in the polypeptide of the present invention is considered to function via protein-protein interaction through the Zn finger domain as well as other polypeptides having the Zn finger domain. Since the Zn finger-like motif of the fln29 polypeptide is significantly homologous to the Zn finger motifs of the TRAF family polypeptides and XAF-1, the fln29 polypeptide is presumably involved in the TNF family signal transduction.

The polypeptide of the present invention can be a native polypeptide or a recombinant polypeptide prepared using genetic recombinant technique. The native polypeptide can be prepared by a method well known in the art, for example, by isolating from human tissues that highly express the polypeptide of the present invention and culture cells derived from humans such as K562 cells and Hela S3 cells by affinity chromatography using an antibody of the present invention described below. The recombinant polypeptide can be prepared by culturing cells transformed with DNA encoding the polypeptide of the present invention described below.

One skilled in the art can also prepare a polypeptide having substantially the same functions as the fln29 polypeptide by modifying amino acids of the fln29 polypeptide, for example, by substitution using a known method. Mutation of amino acids of the polypeptide may occur spontaneously. The polypeptide of the present invention includes variants of the fln29 polypeptide obtainable by substitution, deletion, or addition of amino acids of the polypeptide, which have substantially the same functions as the fln29 polypeptide. As used herein, the term "having substantially the same functions" means that the variants possess biological or/and biochemical activities equivalent to those of the fln29 polypeptide. Such functions are, for example, affinity to other polypeptides, whidh is characteristic of polypeptides having the Zn finger motif. Other polypeptides having affinity to the fln29 polypeptide include the TRAF family polypeptides, the TNF receptor family polypeptides, the IAP family polypeptides, and the XAF1 polypeptide.

Known methods for modifying amino acids are, for example, a method using a site-directed mutagenesis system by PCR (GIBCO-BRL, Gaithersburg, Maryland), a site-directed mutagenesis method by oligonucleotide (Kramer, W. and Fritz, HJ (1987) Methods in Enzymol., 154:350-367), and Kunkel method (Method Enzymol. 85, 2763-2766 (1988)). Amino acid substitution is allowable within usually 10 or less amino acids, preferably 6 or less amino acids, and more preferably 3 or less amino acids. Sites for substitution, deletion, or addition are not limited as long as the polypeptide of the present invention possesses its activities. Such modification should be made in the regions other than the Zn finger-like motif, but the Zn finger-like motif region can be modified as long as the polypeptide of the present invention possesses its activities.

A hybridization technique (Sambrook, J et al., Molecular Cloning 2nd ed. 9.47-9.58, Cold Spring Harbor Lab. press, 1989) is a conventional method for isolating DNA highly homologous to the DNA encoding the fln29 polypeptide from other organisms using DNA encoding the fln29 polypeptide (e.g., SEQ ID NO: 2) or a part thereof, and obtaining a polypeptide substantially having the same functions as the polypeptide of the present invention from the DNA. In other words, one skilled in the art can prepare polypeptides encoded by DNAs that hybridize with the DNA encoding the fln 29 polypeptide and having substantially the same functions as the polypeptide of the present invention. These polypeptides are also included in the polypeptide of the present invention. As used herein, the term "having substantially the same functions" means that polypeptides isolated from other organisms possess biological and/or biochemical activities equivalent to those of the fln29 polypeptide. Such activities include affinity to other polypeptides, which is characteristic of polypeptides having the Zn finger motif. Other polypeptides having affinity to the fln29 polypeptide include the TRAF family polypeptides, the TNF receptor family polypeptides, the IAP family polypeptides and the XAF1 polypeptide. Organisms for isolation of hybridizing DNA are, for example, mice, rats, rabbits, and bovines, and fetal tissues and testis are especially preferable. The hybridization conditions for DNA isolation are as follows. After prehybridization of the test DNAs at 55°C for 30 minutes or more, a labeled probe is added and hybridization is performed by incubating at 37 to 55°C for 1 hour or more. "ExpressHyb Hybridization Solution" (CLONTECH) was used in prehybridization and hybridization. The hybridization products are washed three times in 2xSSC and 0.1% SDS at room temperature for 20 minutes and then once in 1xSSC and 0.1% SDS at 37°C for 20 minutes. The hydridization procedure is preferably performed, after prehybridization at 60°C for 30 minutes or more using "ExressHyb Hybridization Solution" (CLONTECH), a labeled probe is added, hybridization is performed by incubating at 60°C for 1 hour or more, then the hybridization products are washed three times in 2xSSC and 0.1% SDS at room temperature for 20 minutes and twice in 1xSSC and 0.1% SDS at 50°C for 20 minutes. More preferably, prehybridization is performed at 68°C for 30 minutes or more using "ExressHyb Hybridization Solution" (CLONTECH), a labeled probe is added to perform hybridization at 68°C for 1 hour or more, and the hydridization products are washed three times in 2xSSC and 0.1% SDS at room temperature for 20 minutes and twice in 0.1xSSC and 0.1% SDS at 50°C for 20 minutes.

DNA isolated by the hybridization technique described above, which encodes a polypeptide having substantially the same functions as the fln29 polypeptide, usually has high homology to DNA encoding the fln29 polypeptide (e.g., SEQ ID NO: 2). High homology used herein means that the amino acid sequence deduced from the DNA has percent identity of at least 70% or more, preferably at least 80% or more, and more preferably at least 90% or more, to the amino acid sequence encoded by the DNA set forth in SEQ ID NO: 2. Sequence homology can also be determined by the algorism described in the literature (Proc. Natl. Acad. Sci. USA (1983) 80, 726-730).

Alternatively, DNA with high homology to DNA encoding the fln29 polypeptide can be isolated by known gene amplification technology, for example, using a polymerase chain reaction method with oligonucleotide primers, which specifically bind to DNA encoding the fln29 polypeptide (e.g., SEQ ID NO: 2).

The present invention also relates to DNA encoding the polypeptide of the present invention described above. The DNA encoding the polypeptide of the present invention can be cDNA, genomic DNA, or synthetic DNA. The DNA of the present invention can be used to produce the recombinant polypeptide of the present invention as follows. The DNA encoding the polypeptide of the present invention (e.g., DNA set forth in SEQ ID NO: 2) is inserted into an appropriate expression vector. Cells transformed with said vector are cultured, and expressed polypeptide is purified to give the recombinant polypeptide of the present invention. Examples of host cells for producing the recombinant polypeptide are mammalian cells such as COS cells, CHO cells and NIH3T3 cells, insect cells such as Sf9 cells, yeasts, and *Escherichia coli*, etc. Vectors used for expressing the recombinant polypeptide vary depending on hosts, and include those functioning in mammalian cells such as pcDNA3 (Invitrogen) and pEF-BOS (Nucleic Acids. Res. 1990, 18 (17), p5322), those functioning in insect cells such as "BAC-to-BAC baculovirus expression system" (GIBCO BRL), those functioning in yeasts such as "Pichia Expression Kit" (Invitrogen), and those functioning in *E. coli* cells such as pGEX-5X-1 (Pharmacia) and "QIAexpreess system" (Qiagen). These vectors can be introduced into host cells by, for example, calcium phosphate method, DEAE dextran method, a method using cationic lyposome DOTAP (BOEHRINGER MANNHEIM), electroporation method, calcium chloride method, and the like. The recombinant polypeptide can be purified from transformants by an ordinary method, for example, as described in the literature "The Qiaexpressionist handbook, Qiagen, Hilden, Germany".

The DNA of the present invention can also be used in gene therapy for diseases (e.g., cancer, inflammation, immune disorder) attributed to activity abnormality and expression abnormality of the polypeptide of the present invention. When the DNA of the present invention is used in gene therapy, the DNA is incorporated into an adenovirus vector (e.g., pAdexLcw), a retrovirus vector (e.g., pZIPneo) and the like, and the resulting vector is administered to the body by either *ex vivo* or *in vivo* methods. Synthetic antisense DNA can be used for treatment by administering it as such or after being incorporated into the above vectors.

Furthermore, the DNA of the present invention can be utilized as a tool of searching for a compound which regulates its expression. Compounds thus isolated are candidates of drugs for treating or preventing diseases associated with the DNA of the present invention.

One skilled in the art can also easily produce antibodies that bind to the polypeptide of the present invention. The antibody of the present invention be prepared as polyclonal antibody by immunizing small animals such as rabbits with the polypeptide of the present invention to obtain antiserum, applying the serum to an affinity column coupled with the polypeptide of the present invention, and purifying a fraction that specifically recognizes the polypeptide of the present invention by ammonium sulfate precipitation, polypeptide A or polypeptide G column chromatography, DEAE ion exchange chromatography, affinity chromatography using a column coupled with the polypeptide of the present invention, and the like. The antibody of the present invention can be prepared as a monoclonal antibody by immunizing small animals such as mice with the polypeptide of the present invention, excising the spleen from the immunized animal, grinding it into cells, fusing the cells with mouse myeloma cells using a reagent such as polyethylene glycol, and selecting a clone producing an antibody against the polypeptide of the present invention from the resulting hybridoma. The hybridoma is then intraperitoneally transplanted into a mouse, the ascites is collected, and the monoclonal antibody can be purified from the ascites by, for example, ammonium sulfate precipitation, polypeptide A or polypeptide G column chromatography, DEAE ion exchange chromatography, affinity chromatography using a column coupled with the polypeptide of the present invention, and the like. The antibody to be administered to humans should be humanized antibodies or human antibodies to effectively reduce immunogenicity. A humanized antibody can be produced by, for example, a CDRgraft method in which an antibody gene is cloned from monoclonal antibody-producing cells, and its antigen-determination site is transferred to a known human antibody. A human antibody can be prepared by immunizing a mouse whose immune system is replaced by human immune system, followed by the usual procedure to produce monoclonal antibody. The antibody of the present invention thus prepared can be utilized for purification of the polypeptide of the present invention, screening for a compound which regulates activities of the polypeptide of the present invention, detection of tumor markers, and treatment of diseases associated with the polypeptide of the present invention.

In addition, the present invention relates to a method of screening for a compound that binds to the polypeptide of the present invention. The screening method comprises contacting test compounds with the polypeptide of the present invention and selecting compounds that bind to the polypeptide of the present invention. Test compounds include polypeptides and artificially synthesized low-molecular weight compounds.

Polypeptides binding to the polypeptide of the present invention can be isolated by immunoprecipitation using the polypeptide of the present invention. Immunoprecipitation is performed by contacting test compounds, for example, extracts from cells or tissues including organs sich as fetal tissues and adult testis, and culture cells such as K562 cells and Hela S3 cells, with the polypeptide of the present invention, reacting the resulting complex of the polypeptide of the present invention and another polypeptide binding thereto with an antibody to form an immunocomplex, and allowing the complex to precipitate (Experimental Medicine Supplement, New Gene Engineering Technology Handbook, Yodosha, p304-308 (1996)). A compound that binds to the polypeptide of the present invention can also be isolated by applying culture supernatant or extract from cells, which presumably express a polypeptide binding to the polypeptide of the present invention (e.g., organs such as fetal tissues and adult testis, and culture cells such as K562 cells and Hela S3 cells) to an affinity column coupled with the polypeptide of the present invention, and purifying a polypeptide that specifically binds to the affinity column. In addition, DNA encoding the polypeptide that binds to the polypeptide of the present invention can be obtained by analyzing the amino acid sequence of the polypeptide obtained as described above, synthesizing oligo DNA based on the resulting sequence information, and screening a cDNA library using the DNA as a probe.

One skilled in the art can readily screen compounds that bind to the polypeptide of the present invention by other known methods including a method in which a natural bank or a random phage peptide display library is contacted with the polypeptide of the present invention and binding molecules are screened. Substances other than polypeptides such as chemically synthesized compounds that bind to the polypeptide of the present invention can be screened using high-throughput based on combinatorial chemistry technique (Wrighton NC; Farrell FX; Chang R; Kashyap AK; Barbone FP; Mulcahy LS; Johnson DL; Barrett RW; Jolliffe LK; Dower WJ., Small peptides as potent mimetics of the polypeptide hormone erythropoietin, Science (UNITED STATES) Jul 26 1996, 273 p458-64, Verdine GL., The combinatorial chemistry of nature. Nature (ENGLAND) Nov 7 1996, 384 p11-13, Hogan JC Jr., Directed combinatorial chemistry. Nature (ENGLAND) Nov 7 1996, 384 p17-9).

A gene encoding a polypeptide that binds to the polypeptide of the present invention can be directly screened using the polypeptide of the present invention. This screening method comprises contacting a test gene product with the polypeptide of the present invention and selecting a gene corresponding to a gene product bound to the polypeptide of the present invention. Any genes can be used as a test gene without limitation. For example, a cDNA library prepared from desired cells which potentially express a polypeptide that binds to the polypeptide of the present invention is preferable. Specifically, this method can be performed as follows. First, a cDNA library is constructed using phage vector (λgt11, λZAP etc.) from cells which potentially express a polypeptide that binds to the polypeptide of the present invention (e.g., organs such as fetal tissues and adult testis, and culture cells such as K562 cells and Hela S3 cells) and allowed to express on LB-agar plates. Polypeptides expressed on the plates are fixed on filters and reacted with the polypeptide of the present invention, which is biotinylated or purified as a fusion polypeptide with the GST polypeptide. Plaques expressing a polypeptide that binds to the polypeptide of the present invention are detected by streptavidin or by "Westwestern blotting method" using an anti-GST antibody (Skolnik EY, Margolis B, Mohammadi M, Lowenstein E, Fischer R, Drepps A, Ullrich A, and Schlessinger J (1991) Cloning of P13 kinase-associated p85 utilizing a novel method for expression/cloning of target polypeptides for receptor tyrosine kinases. Cell 65, 83-90). "Two-hybrid system" can also be used for the method of the present invention. In this method, the polypeptide of the present invention is expressed in yeast cells as, for example, a fusion polypeptide with SRF binding domain or GAL4 binding domain. A cDNA library is constructed from cells which potentially express a polypeptide that binds to the polypeptide of the present invention so that the polypeptide expresses as a fusion polypeptide with VP16 or GAL4 transcription activation domain. The library is introduced into the above yeast cells. cDNAs derived from the library are isolated from detected positive clones and transformed into *E. coli* cells to express polypeptides (when the polypeptide of the present invention and a polypeptide binding thereto express together in yeast cells, a reporter gene is activated, and positive clones can be detected.) ("MATCHMARKER Two-Hybrid System," "Mammalian MATCHMAKER Two-Hybrid Assay Kit," "MATCHMAKER One-Hybrid System" (these are all produced by CLONTECH), "HybriZAP Two-Hybrid Vector System" (Stratagene), the system described in "Dalton S, and Treisman R (1992) Characterization of SAP-1, a polypeptide recruited by serum response factor to the c-fos serum response element. Cell 68, 597-612"). The isolated polypeptide binding to the polypeptide of the present invention can be utilized as a tool in a screening system to search for compounds which regulate activities of the polypeptide of the present invention. In addition, DNA encoding it can be utilized in the same way as the DNA encoding the polypeptide of the present invention. The isolated compounds binding to the polypeptide of the present invention can also be used as therapeutic or prophylactic agents for diseases (e.g., cancer, inflammation, immune disorder) attributed to activity abnormality and expression abnormalities of the polypeptide of the present invention.

### Brief Description of the Drawings

Figure 1 compares the amino acid sequence encoded by the 2nd frame of EST N29133 (N29133 f2) and the N-terminal Zn finger domain amino acid sequence of mouse TRAF6 (mTRAF6). Asterisks present identical amino acids, and dots, similar amino acids.
Figure 2 shows the amino acid sequence of the fln29 polypeptide. The Zn finger-like motif is underlined, and the Glu stretch is shadowed. The sequence in the box is a site that undergoes tyrosine phosphorylation.
Figure 3 compares the amino acid sequence of the fln29 polypeptide, mouse TRAF6, and the N-terminal region of XAF-1. Asterisks present identical amino acids, and dots, similar amino acids.
Figure 4 shows the Northern blot analysis of various fetal tissues.
Figure 5 shows the Northern blot analysis of various adult tissues.
Figure 6 shows the Northern blot analysis of various carcinoma cell lines.
Figure 7 shows the Western blot analysis of cell extract of COS 7 cells expressing either pcDNA3/fln29HA or control plasmid DNA. The fln29HA polypeptide of approximately 70 kDa is stained with an anti-HA antibody.
Figure 8 shows SDS-PAGE patterns of the GST-fln29 fusion polypeptide purified from transformed *E. coli* cells (Coomassie staining). The fusion polypeptide of about 100 kDa (arrow) is purified.
Figure 9 shows SDS-PAGE patterns of various labeled polypeptides produced by *in vitro* translation (analyzed with BAS2000).
Figure 10 shows a result of *in vitro* binding assay. GST-fln29, GST, and GST-CD30 in the left-hand column are recombinant polypeptides added as probes. The polypeptides are added in the + lane. Labeled polypeptide added shows in the upper. Human TRAF3 (hTRAF3) clearly binds to GST-fln29 used as a probe.

### Best Mode for Carrying out the Invention

The present invention is illustrated in detail below with reference to Examples, but is not to be construed as being limited thereto.

### Example 1 Identification of a novel gene

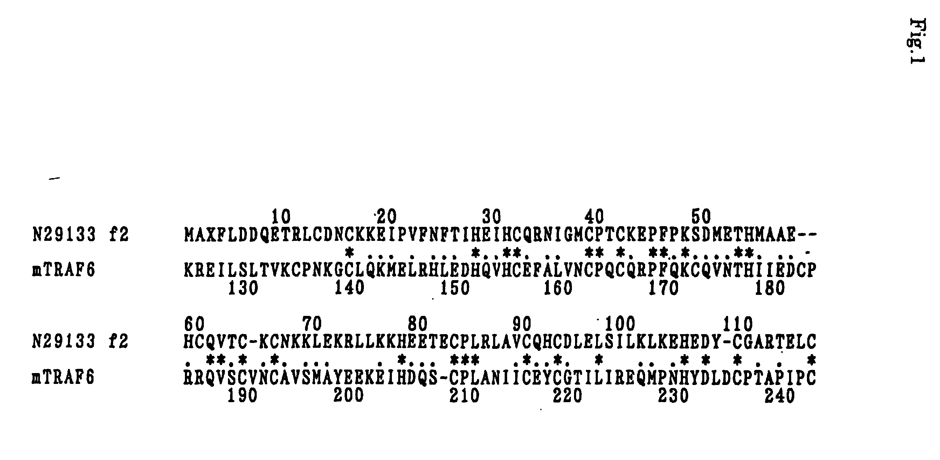

In order to identify a new factor involved in signal transduction of the TNF family, the public EST database was searched based on a known factor sequence (the sequence of mTRAF6 shown in Fig. 1). As a result, EST N29133 encoding open reading frame (ORF) significantly homologous to the Zn finger domain in the N-terminal region of the TRAF family polypeptide was found. The alignment of the amino acid sequence encoded by the 2nd frame of the EST and the mouse TRAF6 amino acid sequence is shown in Fig. 1. Seventeen ESTs that were presumably derived from the same gene as the above EST derived from have been registered in UniGene site (http://www.ncbi.nlm.nih.gov/Schuler/UniGene/) of NCBI (National Center for Biotechnology Information) in WWW, but the entire gene structure was not known. Thus, this gene was further analyzed by cloning cDNA containing the entire ORF by PCR.

### (1) cDNA cloning

On the basis of the sequences of public EST N29133 and EST N21170 that is derived from the 3'-terminus of the same gene as EST N29133 derived from, three primers were designed; N29 S1 (5' CAAGAAGTTGGAGAAGAGGCTGTT 3', SEQ ID NO: 3) and N29 A2 (5' ACCTCCAGCTCAACACATCAAAAG 3', SEQ ID NO: 4) as primers to amplify the region between the two ESTs, and N29 A3 (5' TGGCAACCTCATTTCTCTTTTCCT 3', SEQ ID NO: 5) as a primer to amplify the upstream of the 5' terminus of N29133 by a method of rapid amplification of cDNA ends (RACE).

The region between the two ESTs was amplified by PCR comprising 1 cycle of 94°C for 3 minutes, 35 cycles of 94°C for 30 seconds, 58°C for 1 minute and 72°C for 3 minutes, and 1 cycle of 72°C for 10 minutes or less, using a single-stranded cDNA synthesized from human fetal brain poly(A)⁺RNA as a template.

Separately, the 5' region was amplified by PCR comprising 1 cycle of 94°C for 2 minutes, 5 cycles of 94°C for 30 seconds and 68°C for 4 minutes, 30 cycles of 94°C for 30 seconds, 62°C for 1 minute, and 72°C for 3 minutes, and 1 cycle of 72°C for 10 minutes or less, using "Marathon™ Ready cDNA" (CLONTECH) (Chenchik, A., Moqadam, F., and Siebert, P (1995) CLONTECHniques X, 5-8) derived from the human fetal brain as a template.

The above PCR was carried out by a hot start method (Kellogg, D.E., et al. (1994) Bio Techniques 16, 1134-1137) with "TaqStart™ Antibody" (CLONTECH) using "TaKaRa Ex Taq" (TaKaRa) as a polymerase. After the reaction, the resulting bands were recovered using "QIAquick Gel Extraction Kit" (QIAGEN), and the recovered band DNA was subcloned into "pT7Blue-T vector" (Novagen) by an ordinary TA cloning method.

### (2) Decoding of sequence

The subcloned DNA was sequenced by cycle sequencing method with "ABI PRISM™ Dye Terminator Cycle Sequencing Ready Reaction Kit With AmplyTaq DNA Polymerase, FS" using a plasmid DNA prepared by an alkali SDS method or a colony PCR product as a template. The sequence were decoded with "ABI 377 DNA Sequencer."

A colony containing a transformant was directly suspended in PCR reaction solution containing vector primers, M13 P4-22 (5' CCAGGGTTTTCCCAGTCACGAC 3', SEQ ID NO: 6) and M13 P5-22 (5' TCACACAGGAAACAGCTATGAC 3', SEQ ID NO: 7) to perform Colony PCR. After the PCR reaction, an amplified insert DNA separated from non-reacted primers and nucleotides, etc. by gel filtration was used as a template for sequencing.

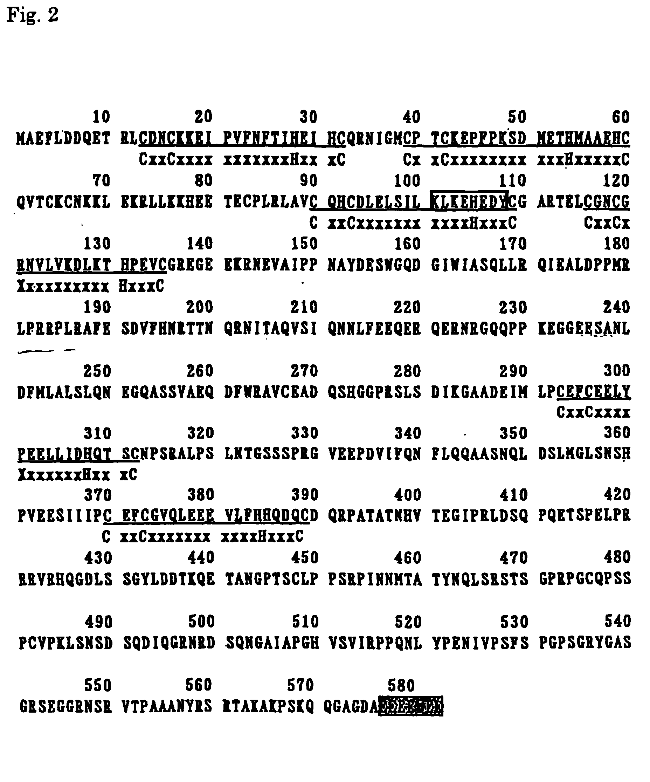

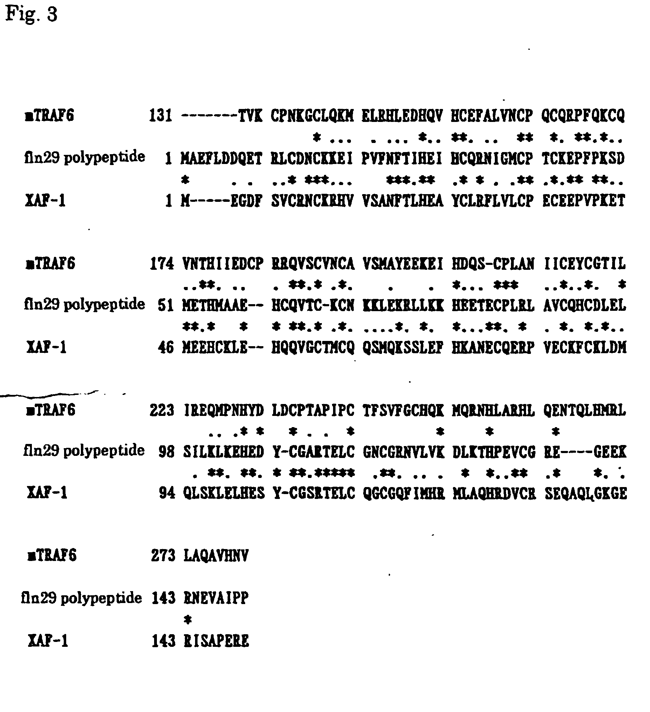

The gene (named "fln29") was confirmed to encode ORF of 582 amino acid residues. The nucleotide sequence is set forth in SEQ ID NO: 2, and the amino acid sequence deduced from the nucleotide sequence is set forth in SEQ ID NO: 1 and in Fig. 2. The amino acid sequence contains six Zn finger-like motif repeats consisting "Cys-Xaa(2)-Cys-Xaa(11)-His-Xaa(3 or 5)-Cys (Xaa represents any amino acid)" (Fig. 2), and the homologous region to TRAF corresponds to the region of the first four Zn finger-like domains (Fig. 3). However, neither the Ring finger domain nor the C-terminal domain (the TRAF-N domain, the TRAF-C domain) which commonly exists in TRAF family polypeptides was found in the sequence, indicating that the fln29 polypeptide does not belong to the TRAF family although it has the homologous domain (Takeuchi, M. et al., (1996) J. Biol. Chem. 271, 19935-19942).

The Zn finger-like domain of the fln29 polypeptide was also demonstrated to be highly homologous to XAF-1 (XIAP associated factor-1) (Fig. 3); homology to XAF-1 was found in a wider and longer range than that to the TRAF family polypeptides. Since XAF-1 presumably inhibits apoptosis via XIAP, regulates inflammation and immune response via transcription factor NF-κB, and regulates cell cycle and cell proliferation, the fln29 polypeptide may also be involved in these functions.

The fln29 polypeptide has such structural characteristics other than the Zn finger-like motif as six Glu stretches at the C-terminus and the site that can undergo tyrosine phosphorylation (at the positions 101-108). Any other specific motif structure, hydrophobic regions such as a transmembrane region, intracellular localization signal or the like were not found. The theoretical isoelectric point (pI) was approximately 5.

### Example 2 Northern blot analysis

The fragment amplified using the primers of N29 S1 and N29 A2 was labeled with [α-³²P]dCTP by a random primer method using "Ready-to Go DNA labeling beads" (Pharmacia), and used as a probe for Northern blot analysis. Hybridization was performed in "ExpressHyb Hybridization Solution" (CLONTECH) at 68°C using "Multiple Tissue Northern (MTN) Blot-Human, Human II, Human III, Human Fetal II, Human Cell Line" (CLONTECH) following the manufacturer's manual. Final washing was performed with 0.1xSSC and 0.1% SDS at 50°C.

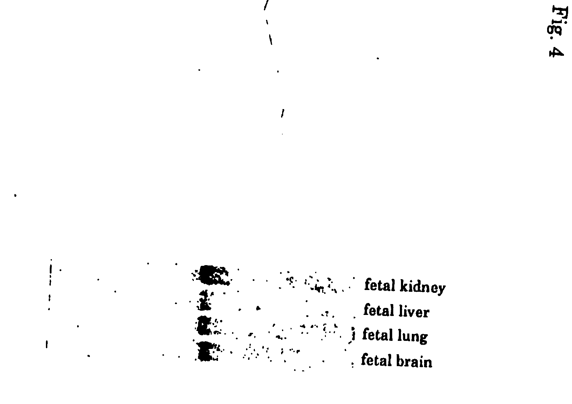

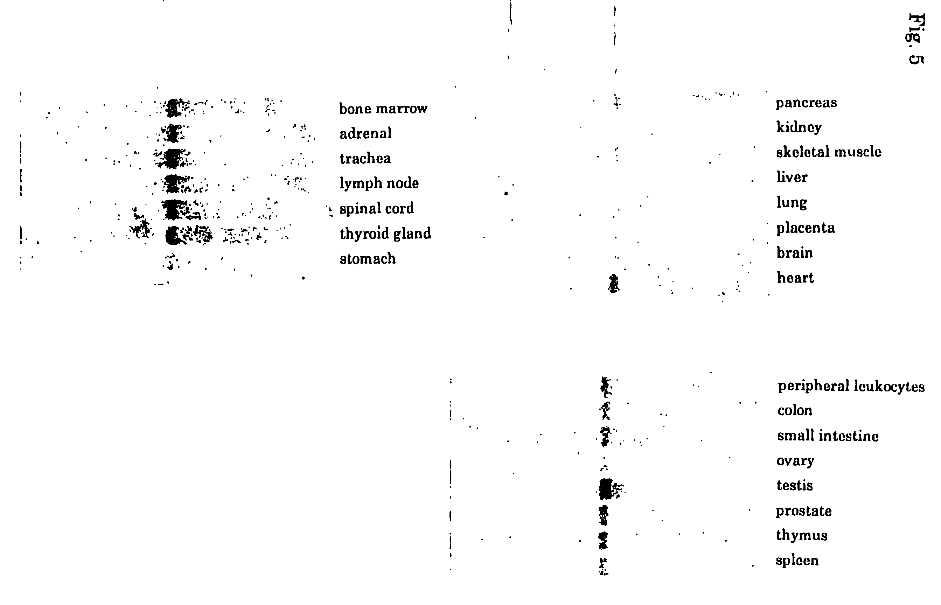

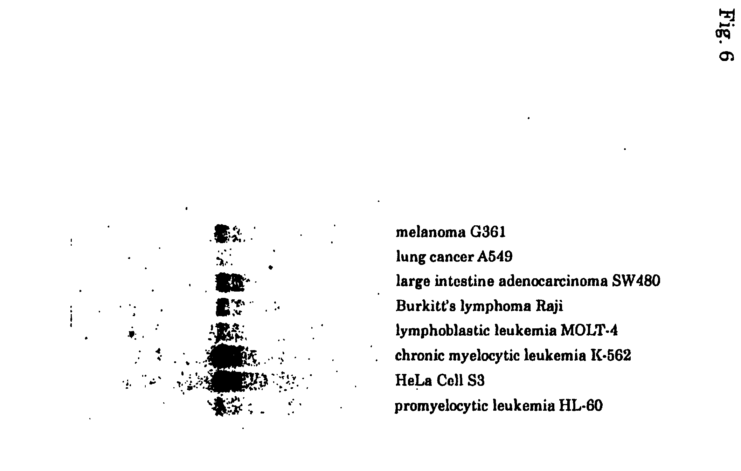

Northern blot analysis revealed that the gene weakly expressed in all tissues but more intensively in fetal tissues (especially brain and kidney) and adult testis (Figs. 4 and 5). These tissues contain more undifferentiated cells, rapidly growing cells, and cells which are removed by apoptosis during differentiation compared with other tissues. The gene is strongly expressed in various carcinoma cell lines (especially K562 cells and Hela S3 cells shown in Fig. 6). These results indicate that the gene can be involved in apoptosis and mechanism of regulating differentiation, and further in canceration through these functions.

### Example 3 Construction of fln29 polypeptide expression vector

The fln29 cDNA containing the entire coding region was amplified by PCR using FLN29 WSB2 primer (5' GATGGATCCTGGAAGAGCTAAAGATGGCTGAAT 3', SEQ ID NO: 8) and FLN29 WAB2 primer (5' GATGGATCCGAAGACAGGAACCGATGTAAAGTC 3', SEQ ID NO: 9) using a cDNA synthesized from human fetal brain poly A⁺ RNA (CLONTECH) as a template. PCR was performed by 1 cycle of 94°C for 3 minutes, 35 cycles of 98°C for 15 seconds and 68°C for 30 seconds, and 1 cycle of 72°C for 10 minute using KOD polymerase (TOYOBO) as a DNA polymerase. The resulting DNA fragment of about 1.8 kbp was digested with BamHI. The digestion product was purified by agarose gel electrophoresis and ligated to the BamHI site of a mammalian expression vector pcDNA3 (Invitrogen). A clone having a correst sequence was selected by sequencing, which was named pcDNA3/fln29w. Then, cDNA encoding the amino acid sequence with HA epitope tag sequence (NH2 Tyr-Pro-Tyr-Asp-Val-Pro-Asp-Tyr-Ala-Ser-Lue COOH) at the C-terminus was amplified by PCR using FLN29 HA1 primer 
(5' GATGGGCCCTTACAGGGAGGCATAGTCAGGCACATCATATGGGTACTCCTCCTCTTCCTCT
TCTGCATC 3', SEQ ID NO: 10)
 and FLN29 HA2 primer (5' CAGGGAGACCTGTCTTCTGGTTACCTG 3', SEQ ID NO: 11) and using pcDNA3/fln29w as a template. The amplified fragment was digested with ApaI and ligated to pcDNA3/fln29w from which a fragment cut out by digestion with ApaI was removed. A clone having a correct sequence was selected and named pcDNA3/fln29HA.

In addition, plasmid DNA for expression of the fln 29 polypeptide in *E. coli* was prepared as follows. cDNA containing the entire coding region of the fln29 polypeptide with an appropriate frame for *E. coli* expression vector pGEX-5X-1 (Pharmacia) was amplified by PCR using N29 GST2 primer (5' GATGGATCCGAAGACAGGAACCGATGTAAAGTC 3', SEQ ID NO: 12) and N29 GST3 primer (5'GATGGATCCCCATGGCTGAATTTCTAGATGACCAG 3', SEQ ID NO: 13) and using pcDNA3/fln29w as a template. The PCR product was digested with BamHI, and the digested PCR product was ligated into the BamHI site of pGEX-5X-1 vector. A clone having a correct sequence was selected and named pGEX/fln29w.

### Example 4 Expression of the fln29 polypeptide in mammalian cells

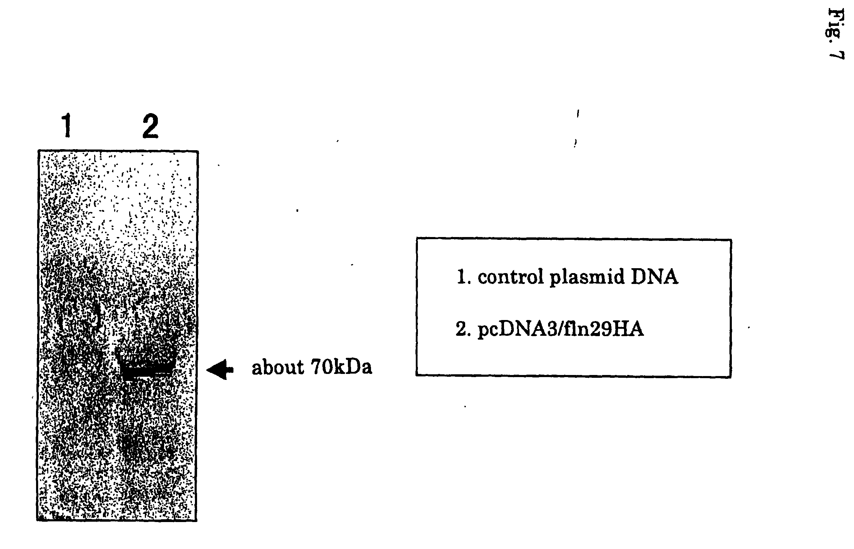

About 7.5 µg of pcDNA3/fln29HA plasmid DNA or control plasmid DNA was transfected to COS7 cells by a method using cationic phospholipid DOTAP (BOEHRINGER MANNHEIM). First, about 10⁶ COS7 cells were sown on a 10 cm-dish and incubated overnight. A mixture of 7.5 µg of plasmid DNA and 45 µl of DOTAP was added thereto, and the cells were incubated for about 3 hours. The medium was then replaced with fresh one, and the cells were further cultured for two days. The cells were recovered by treatment with trypsin-EDTA solution, washed with PBS once, and lysed with NP40 lysis buffer (10 mM Tris-HCl pH 7.8, 1% NP40, 0.15 M NaCl, 1 mM EDTA, 10 µg/ml aprotinin, 1 mM PMSF). Cell extract was obtained by centrifugation and separated on SDS-gel electrophoresis, followed by Western blotting analysis using an anti-HA antibody (SANTA CRUZ). Approximately 70 kDa band specifically appeared when pcDNA3/fln29HA plasmid DNA was transfected to the cells, indicating that the fln29 polypeptide was expressed (Fig. 7).

### Example 5 Expression of the fln29 polypeptide in E. coli

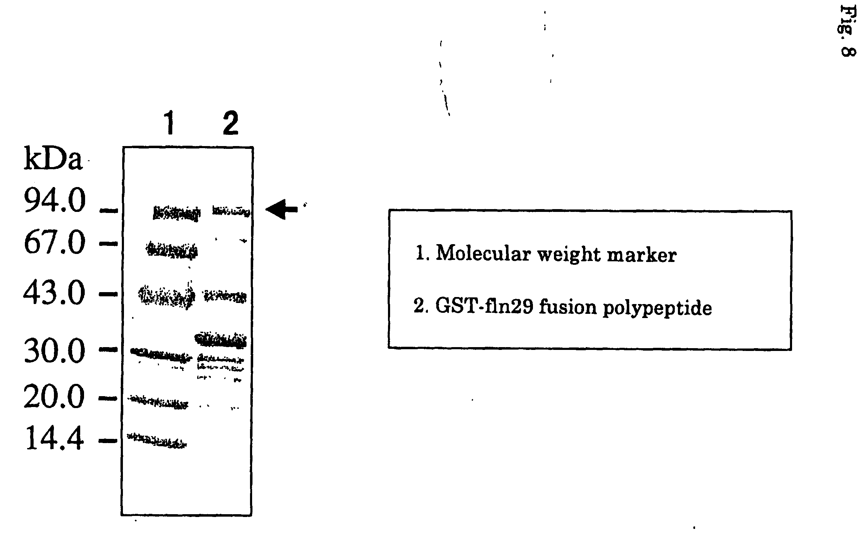

The fln29 polypeptide fused to the glutathione S transferase (GST) polypeptide was expressed in *E. coli* cells and was purified. First, *E. coli* DH5α cells carrying pGEX/fln29w plasmid DNA were cultured in 10 ml of 2xYT medium at 37°C overnight. A portion of the culture was diluted 100-fold with fresh 2xYT medium and continuously cultured at 37°C until the optical density at 600 nm reached 0.6. Then, isopropyl-β-D(-)-thiogalactoprynoside (IPTG) was added to a final concentration of 0.1 mM, and the cells were cultured for several hours. The cells were collected by centrifugation, suspended in a mixture of 1% Triton X-100, 1% Tween 20, and PBS, and sonicated to disrupt the cells and solubilize polypeptides. The fln29 polypeptide expressed as the fusion polypeptide to the GST polypeptide was affinity purified using glutathione Sepharose 4B (Pharmacia) from the solubilized sample. The purified polypeptide was subjected to SDS-PAGE and stained with Coomassie (CBB). The result is shown in Fig. 8. It is clearly demonstrated that the GST-fln29 fusion polypeptide of approximate 100 kDa was purified, although degenerated products were detected.

### Example 6 In vitro binding test

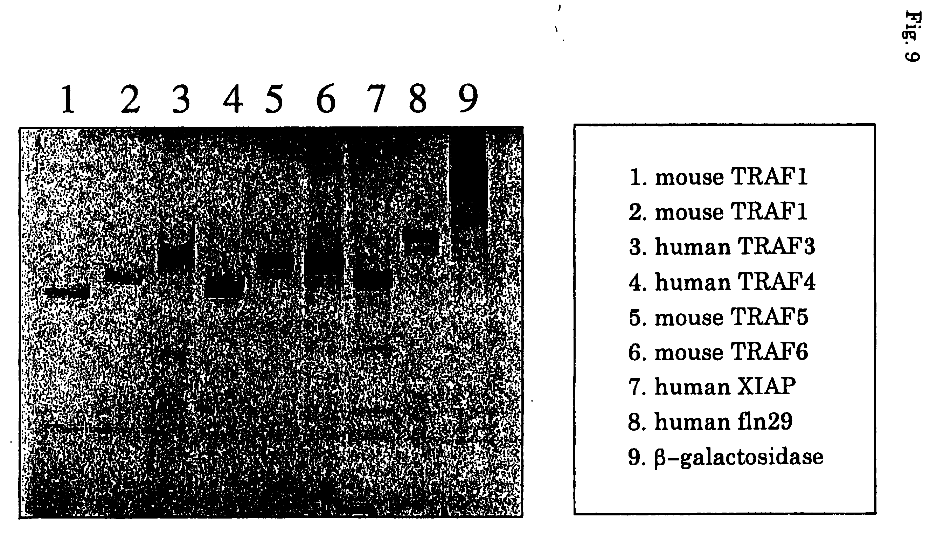

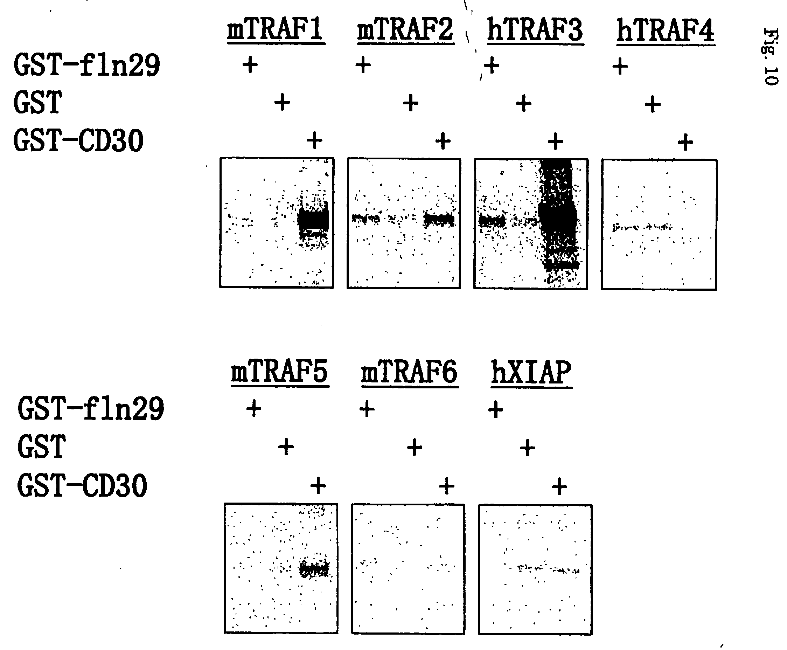

*In vitro* translation were carried out using expression plasmid DNA of mouse TRAF1, mouse TRAF2, human TRAF3, human TRAF4, mouse TRAF5, mouse TRAF6, and human XIAP polypeptides as templates in the presence of [³⁵S]methionine and using Single Tube Polypeptide System 2, T7 (Novagen) following the manufacturer's manual, and each [³⁵S]-labeled polypeptide was obtained (Fig. 9). About 5 µg each of the GST-fln29 fusion polypeptide described above expressed in *E. coli* cells, the GST polypeptide as a negative control, and the GST-CD30 polypeptide (intracellular region) as a positive control were bound to glutathione Sepharose beads, and mixed with an appropriate amount of the above labeled polypeptide in binding buffer (50 mM Tris-HCl pH 7.4, 150 mM NaCl, 10% glycerol, 0.1% NP40, 1 mM DTT, 1mM PMSF, 10 µg/ml aprotinin). The resulting mixture was stirred at 4°C for 2 hours, and centrifuged to precipitate the polypeptide complex formed with beads. The precipitates was washed with binding buffer five times to remove non-specifically binding polypeptides. The polypeptides finally precipitated were applied to SDS-PAGE and analyzed with BAS2000 Analyzer (Fuji Film). The result demonstrated that the fln29 polypeptide was bound to TRAF3 (Fig. 10), suggesting that the fln 29 polypeptide binds to TRAF3 in cells, and plays an important role in signal transduction of immune responses, cell proliferation and cell differentiation in which TRAF3 is involved.

### Industrial Applicability

The present invention provides a novel polypeptide having the Zn finger-like motif and DNA encoding said polypeptide. The polypeptide of the present invention is presumably involved in apoptosis, canceration, inflammatory responses, and immune responses. For the sake of it, the polypeptide of the present invention can be used as a tool for screening for candidate low molecular compounds for therapeutic or prophylactic drugs for cancer, inflammation, immune disorders and the like. Since the Zn finger domain of the polypeptide of the present invention is assumed to interact with other polypeptides, the polypeptide of the present invention is useful for screening for interacting polypeptides and genes encoding them. Furthermore, the polypeptide of the present invention can be used as a diagnostic reagent for above diseases, and as antigen for preparing antibodies that regulate activities of the polypeptide of the present invention. The DNA encoding the polypeptide of the present invention can be used in not only production of the polypeptide of the present invention but also gene therapy and gene diagnosis of the above diseases.

## Claims

1. A polypeptide comprising the amino acid sequence set forth in SEQ ID NO:1, or a polypeptide comprising said sequence in which one or more amino acids are substituted, deleted, or added, and having substantially the same functions as the polypeptide comprising the amino acid sequence set forth in SEQ ID NO:1.

2. A polypeptide encoded by DNA that hybridizes with DNA set forth in SEQ ID NO:2 and having substantially the same functions as a polypeptide comprising the amino acid sequence set forth in SEQ ID NO:1.

3. A DNA encoding the polypeptide of claim 1.

4. A DNA hybridizing with the DNA set forth in SEQ ID NO:2 and encoding a polypeptide having substantially the same functions as a polypeptide comprising the amino acid sequence set forth in SEQ ID NO:1.

5. A vector comprising the DNA of claims 3 or 4.

6. A transformant harboring the DNA of claims 3 or 4.

7. A method of producing the polypeptide of claim 1 or 2, the method comprising culturing the transformant of claim 6.

8. An antibody that binds to the polypeptide of claims 1 or 2.

9. A method of screening for a compound that binds to the polypeptide of claims 1 or 2, the method comprising contacting a test compound with the polypeptide of claims 1 or 2 and selecting a compound bound to the polypeptide of claims 1 or 2.

10. The method of claim 9, wherein a compound is a polypeptide.

11. A method of screening for a gene encoding a polypeptide that binds to the polypeptide of claims 1 or 2, the method comprising contacting a test gene product with the polypeptide of claims 1 or 2, and selecting a gene corresponding to a gene product that binds to the polypeptide of claims 1 or 2.

12. A compound that binds to the polypeptide of claims 1 or 2.

13. The compound of claim 12, wherein the compound is a polypeptide.

14. The compound of claim 12, wherein the compound is isolable by the method of claim 9.

15. The compound of claim 13, wherein the compound is isolable by the method of claim 10.

16. A gene encoding a polypeptide that binds to the polypeptide of claims 1 or 2.

17. The gene of claim 16, wherein the gene is isolable by the method of claim 11.
